# EUROPÄISCHE PATENTSCHRIFT

(11) **EP 3 084 425 B1**
(45) Veröffentlichungstag und Bekanntmachung des Hinweises auf die Patenterteilung: **27.09.2017**
(21) Anmeldenummer: 14821483.6
(22) Anmeldetag: 19.12.2014
(51) Int. Cl.: G01N 33/36

(54) **VORRICHTUNG ZUR VERKNÄUELUNG UND ZUM WÄGEN VON GARN**
DEVICE FOR FORMING A BALL OF YARN AND WEIGHING YARN
DISPOSITIF POUR LE PELOTAGE ET LE PESAGE DE FIL

(30) Priorität: 20.12.2013 CH 21162013
(43) Veröffentlichungstag der Anmeldung: 26.10.2016
(73) Patentinhaber: Uster Technologies AG, 8610 Uster (CH)
(72) Erfinder: KUSTER, Martin, CH-8733 Eschenbach (CH); PIRANI, Peter, CH-8624 Grüt (CH); SCHEIBER, Patrik, CH-8155 Niederhasli (CH)
(74) Vertreter: Pliska, Pavel
(86) Internationale Anmeldenummer: PCT/CH2014/000180
(87) Internationale Veröffentlichungsnummer: WO 2015/089681

(56) Entgegenhaltungen:
- WO-A1-89/03531
- DE-A1- 1 940 291

## Beschreibung

### FACHGEBIET

Die vorliegende Erfindung liegt auf dem Gebiet der Prüfung von Garn. Sie betrifft eine Vorrichtung zur Verknäuelung von Garn und eine Vorrichtung zum Wägen von Garn, gemäss den Oberbegriffen der betreffenden Patentansprüche.

### STAND DER TECHNIK

Zur Bestimmung der Garnnummer (Garnmasse pro Länge oder Garnlänge pro Masse) wird eine bestimmte Länge des zu prüfenden Garns abgemessen, vom Rest des Garns getrennt und gewogen. Zum Wägen muss der Garnabschnitt auf den Teller einer Präzisionswaage gebracht werden, was vorteilhafterweise in Form eines Garnknäuels geschieht.

Ein Modul zur Bestimmung der Garnnummer ist in der US-5,025,660 A beschrieben. In diesem Modul erfolgt die Knäuelbildung in einem kreissymmetrischen Behälter, der aus einer glockenförmigen Kappe und einem plattenförmigen Wägeteller besteht. Das Garn wird durch ein Rohr, das tangential von schräg oben in die Kappe mündet, mit einer Luftströmung in die Kappe hinein geblasen. Im Hohlraum, der nach unten durch den Wägeteller und seitlich durch die Kappe begrenzt ist, bildet sich eine Luftströmung, die das Garn zu einem kugelförmigen Knäuel verknäuelt. Die eingeblasene Luft entweicht durch eine Öffnung am oberen Ende der Kappe. Wenn die abgemessene Garnlänge erreicht ist, bleibt der Knäuel auf dem Wägeteller liegen. Dieser wird auf die Waage abgesenkt, worauf der Garnknäuel gewogen wird. Danach wird der Knäuel vom Wägeteller weggeblasen, und der Vorgang kann wiederholt werden. Ein Nachteil dieser Vorrichtung aus dem Stand der Technik besteht darin, dass die Kappe und der Wägeteller beweglich sein müssen. Dadurch entstehen höhere Herstellungskosten und eine gewisse Fehleranfälligkeit im Betrieb.

### DARSTELLUNG DER ERFINDUNG

Es ist deshalb eine Aufgabe der vorliegenden Erfindung, die Nachteile der aus der US-5,025,660 A bekannten Vorrichtung zu vermeiden. Insbesondere sollen bewegliche Teile vermieden werden.

Diese und andere Aufgaben werden durch die erfindungsgemässen Vorrichtungen, wie sie in den unabhängigen Patentansprüchen definiert sind, gelöst. Vorteilhafte Ausführungsformen sind in den abhängigen Patentansprüchen angegeben.

Die Erfindung beruht auf der Idee, einen starren oder statischen Behälter für die pneumatische Verknäuelung von Garn durch eine Luftströmung zur Verfügung zu stellen. Der Behälter hat ein nach aussen begrenztes Innenvolumen. Die Begrenzung stellt sicher, dass das Garn im Innenvolumen bleibt und dort durch die Luftströmung verknäuelt wird. Ferner soll das Innenvolumen eine nach unten offene Austrittsöffnung aufweisen, durch welche einerseits die Luft entweichen kann und andererseits das Garnknäuel aus dem Innenvolumen auf eine Waage fällt. Ein unterer Teil einer das Innenvolumen begrenzenden Innenwand ist zur Austrittsöffnung hin nach unten geneigt, um bei abgestellter Luftströmung ein Rollen und/oder Gleiten des Garnknäuels zur Austrittsöffnung hin zu bewirken.

Wenn in dieser Schrift von einem "starren" Behälter, oder "starren" Innenwand etc. die Rede ist, so ist damit gemeint, dass die genannten Gegenstände keine gegeneinander beweglichen Teile aufweisen. Die starren Gegenstände können einstückig oder aus mehreren fest zusammengefügten Teilen hergestellt sein. Im letzteren Fall kann auch von einer "statischen Baugruppe" gesprochen werden.

Die in dieser Schrift verwendeten Begriffe "oben", "unten", "horizontal", "vertikal" etc. beziehen sich auf das Gravitationsfeld der Erde, dem die Vorrichtung im betriebsbereiten Zustand ausgesetzt ist.

Das Innenvolumen soll in vertikaler Richtung durch eine Innenwand teilweise nach aussen begrenzt sein. Damit ist Folgendes gemeint: Wenn man sich von gewissen Punkten im Innenvolumen aus in vertikaler Richtung bewegt, stösst man auf die Innenwand; es gibt hingegen andere Punkte im Innenvolumen, von denen aus eine Bewegung in vertikaler Richtung nach aussen führt, ohne Behinderung durch die Innenwand. Ausserdem soll das Innenvolumen in horizontaler Richtung durch die Innenwand vollständig nach aussen begrenzt sein. Das bedeutet, dass jede Bewegung in horizontaler Richtung von irgendeinem Punkt im Innenvolumen aus auf die Innenwand stösst - mit Ausnahme einer Eintrittsöffnung für das Garn.

Die erfindungsgemässe Vorrichtung zur Verknäuelung eines Garns beinhaltet einen Behälter mit einem Innenvolumen, welches in vertikaler Richtung durch eine Innenwand teilweise sowie in horizontaler Richtung durch die Innenwand vollständig nach aussen begrenzt ist. In der Innenwand ist eine Eintrittsöffnung angebracht, durch welche das Garn mit einer Luftströmung in das Innenvolumen einschiessbar ist. Die Innenwand ist starr ausgebildet. Das Innenvolumen weist eine nach unten offene Austrittsöffnung für einen im Innenvolumen gebildeten Garnknäuel auf. Ein unterer Teil der Innenwand ist entlang des gesamten Umfangs der Austrittsöffnung zur Austrittsöffnung hin nach unten geneigt.

In einer Ausführungsform ist ein Neigungswinkel des unteren Teils der Innenwand gegen die Horizontale nicht grösser ist als -10°. Er liegt bspw. zwischen -30° und -10° und beträgt vorzugsweise -15°.

Das Innenvolumen kann um eine vertikale Achse kreissymmetrisch sein. Unter dem Begriff "Kreissymmetrie" wird in dieser Schrift die Rotationssymmetrie bezüglich eines beliebigen Winkels verstanden. In einer Ausführungsform hat die Innenwand im Wesentlichen die Form eines Teils eines eingebetteten Torus, der durch einen in einer horizontalen Ebene liegenden Kreis definiert ist und dessen Achse vertikal verläuft, der nach innen offen ist und dessen unterer Teil zur Austrittsöffnung hin nach unten geneigt ist. Ein eingebetteter Torus ist die Menge aller Punkte, die vom genannten Kreis einen festen Abstand haben, wobei der feste Abstand kleiner als der Kreisradius ist.

In einer Ausführungsform weist der Behälter eine zentrale, nach oben offene Öffnung auf, welche ein Ausströmen von Luft aus dem Innenvolumen nach oben ermöglicht. Die Fläche der nach oben offenen Öffnung ist vorzugsweise grösser als die Fläche der Austrittsöffnung.

In einer Ausführungsform ist die Eintrittsöffnung derart gestaltet und angeordnet, dass das Garn durch die Eintrittsöffnung im Wesentlichen tangential zur Innenwand in das Innenvolumen einschiessbar ist. Die Eintrittsöffnung kann derart gestaltet und angeordnet sein, dass das Garn durch die Eintrittsöffnung in einer nach oben geneigten Richtung in das Innenvolumen einschiessbar ist. Der Neigungswinkel beträgt z. B. 5° bis 25° und vorzugsweise 15° gegen die Horizontale.

Beim Eintritt in das Innenvolumen ist die Strömungsgeschwindigkeit der Luft vorzugsweise grösser als die Einschussgeschwindigkeit des Garns, z. B. mindestens 1.5-mal so gross.

In einer Ausführungsform ist die Innenwand aus einem dünnen Material gebildet, das auch eine Aussenwand des Behälters bildet. Ein Eintrittsstutzen, der in die Eintrittsöffnung mündet, kann mit der Aussenwand starr verbunden sein. Das die Innenwand und die Aussenwand bildende Material ist z. B. ein Metallblech, vorzugsweise Stahlblech oder Aluminiumblech.

Die Innenwand kann mindestens einen in das Innenvolumen hinein ragenden Vorsprung aufweisen. Durch eine solche Schikane wird die Bildung einer laminaren Luftströmung entlang der Innenwand gestört, so dass das eingeschossene Garn einen "chaotischen" Knäuel und nicht etwa einen ringförmigen Wickel bildet. Der mindestens eine Vorsprung kann starr oder flexibel ausgebildet sein. Falls mehrere Vorsprünge vorhanden sind, können sie regelmässig oder unregelmässig auf der Innenwand verteilt sein. Sie können untereinander identisch oder unterschiedlich sein; so ist etwa eine Kombination von starren und flexiblen Vorsprüngen möglich.

Die erfindungsgemässe Vorrichtung zum Wägen eines Garns beinhaltet die oben beschriebene Vorrichtung zur Verknäuelung eines Garns und eine Waage zum Wägen eines durch die Verknäuelung gebildeten Garnknäuels. Die Waage ist unterhalb der Austrittsöffnung angebracht.

In einer Ausführungsform beinhaltet die Vorrichtung zusätzlich eine Luftdüse zum Wegblasen des Garnknäuels von der Waage.

In einer Ausführungsform beinhaltet die Vorrichtung zusätzlich Mittel zur Steuerung des Messverfahrens und zur Auswertung der Messresultate.

Dank der starren Bauweise der erfindungsgemässen Vorrichtung zur Verknäuelung von Garn entfallen mögliche Probleme mit beweglichen Teilen. Die Vorrichtung ist einfacher aufgebaut und kostengünstiger herstellbar als diejenige gemäss dem Stand der Technik.

### AUFZÄHLUNG DER ZEICHNUNGEN

Nachfolgend wird eine bevorzugte Ausführungsform der Erfindung anhand der Zeichnungen erläutert.
- Figur 1: zeigt eine Ausführungsform der erfindungsgemässen Vorrichtung zur Verknäuelung von Garn (a) in einer perspektivischen Ansicht, (b) in einer Draufsicht und (c) in einem Längsschnitt.
- Figur 2: zeigt schematisch Querschnitte von verschiedenen Ausführungsformen der erfindungsgemässen Vorrichtung zur Verknäuelung von Garn.
- Figur 3: zeigt schematisch Draufsichten von verschiedenen Ausführungsformen der erfindungsgemässen Vorrichtung zur Verknäuelung von Garn.
- Figur 4: zeigt eine weitere Ausführungsform der erfindungsgemässen Vorrichtung zur Verknäuelung von Garn (a) in einer teilweise offen gelegten perspektivischen Ansicht und (b) in einer Draufsicht.
- Figur 5: zeigt eine erfindungsgemässe Vorrichtung zum Wägen von Garn (a) in einer perspektivischen Ansicht, (b) in einer Seitenansicht und (c) in einem Längsschnitt.

### AUSFÜHRUNG DER ERFINDUNG

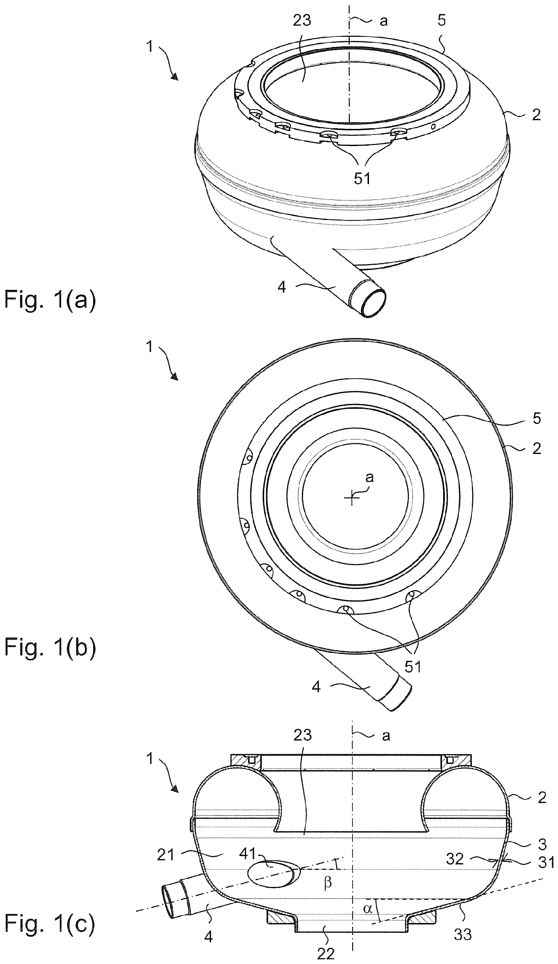

In der **Figur 1** ist eine Ausführungsform der erfindungsgemässen Vorrichtung 1 zur Verknäuelung von Garn (a) in einer perspektivischen Ansicht, (b) in einer Draufsicht bzw. (c) in einem Längsschnitt dargestellt. Die Vorrichtung 1 bildet eine starre Einheit in dem Sinn, dass sie keine gegeneinander beweglichen Teile aufweist. Sie kann somit als eine statische Baugruppe bezeichnet werden.

Die Vorrichtung 1 beinhaltet einen Behälter 2 mit einem um eine vertikale Achse a kreissymmetrischen Innenvolumen 21. In der Ausführungsform der Figur 1 hat der Behälter 2 im Wesentlichen die Form eines Teils eines eingebetteten Torus. Der Behälter 2 wird im vorliegenden Ausführungsbeispiel durch eine dünne Wand 3, bspw. aus einem Metallblech, vorzugsweise Stahlblech oder Aluminiumblech, gebildet. Die Wand 3 definiert somit eine Innenwand 31 im Inneren des Behälters 2 und eine Aussenwand 32. Der eingebettete Torus wird durch einen in einer horizontalen Ebene liegenden Kreis definiert, dessen Mittelpunkt auf der Achse a liegt. Das Innenvolumen 21 ist nach oben geschlossen und nach innen, d. h. zur Achse a hin, offen. Das Innenvolumen 21 weist eine zentrale, nach unten offene Austrittsöffnung 22 für einen im Innenvolumen 21 gebildeten Garnknäuel. Ebenso weist der Behälter 2 eine zentrale, nach oben offene Öffnung 23 auf. Der Aussendurchmesser des Behälters 2 beträgt bspw. ca. 270 mm, sein Innendurchmesser ca. 260 mm, seine Höhe ca. 110 mm, der Durchmesser der Austrittsöffnung 22 ca. 90 mm und der Durchmesser der oberen Öffnung 23 ca. 125 mm.

Ein unterer Teil 33 der Innenwand 31 ist entlang des gesamten Umfangs der Austrittsöffnung 22 zur Austrittsöffnung 22 hin nach unten geneigt. Die Neigung sollte mindestens so gross sein, dass ein Garnknäuel auf dem geneigten Teil 33 nicht ruhen kann, sondern zwangsläufig aufgrund der Gravitationskraft schräg nach unten und zur Austrittsöffnung 22 hin rollt und/oder gleitet und schliesslich durch die Austrittsöffnung 22 das Innenvolumen 21 verlässt. Versuche haben ergeben, dass ein Neigungswinkel α von - 10° gegen die Horizontale nicht überschritten werden sollte, d. h. es sollte gelten: |α| ≥ 10°. Beispielhafte Neigungswinkel α betragen -30° bis -10° und vorzugsweise ca. -15° gegen die Horizontale. Der Neigungswinkel α kann sich entlang des Umfangs der Austrittsöffnung 22 ändern.

In der Wand 3 des Behälters 2 befindet sich eine Eintrittsöffnung 41. Mit der Aussenwand 32 ist ein Eintrittsstutzen 4, der in die Eintrittsöffnung 41 mündet, starr verbunden. Der Innendurchmesser des Eintrittsstutzens 4 beträgt bspw. 20-30 mm und vorzugsweise ca. 25 mm. Auch der Eintrittsstutzen 4 kann aus Stahl bzw. Aluminium gefertigt sein. An den Eintrittsstutzen 4 kann ein (nicht eingezeichneter) Zuführschlauch angeschlossen werden, wie er aus der US-5,025,660 A bekannt ist. Durch den Eintrittsstutzen 4 und die Eintrittsöffnung 41 wird Garn mit einer Luftströmung in das Innenvolumen 21 eingeschossen. Der Eintrittsstutzen 4 und die Eintrittsöffnung 41 sind so gestaltet und angeordnet, dass das Garn im Wesentlichen tangential zur Innenwand 31 in das Innenvolumen 21 einschiessbar ist. Das heisst, dass die durch den Eintrittsstutzen 4 und die Eintrittsöffnung 41 vorgegebene Richtung an der Innenwand 31 ohne abrupte Änderung und ohne Hindernisse fortgesetzt wird. In der hier dargestellten Ausführungsform ist die die durch den Eintrittsstutzen 4 und die Eintrittsöffnung 41 vorgegebene Richtung nach oben geneigt, und zwar um einen Winkel β von 5°-25° und vorzugsweise von ca. 15° gegen die Horizontale.

Im Betrieb wird Garn mit einer Luftströmung durch den Eintrittsstutzen 4 und die Eintrittsöffnung 41 in das Innere des Torusteils eingeschossen. Die Einschussgeschwindigkeit des Garns kann bspw. zwischen 0.5-15 m/s betragen, der Volumenstrom der Luft bspw. 1-20 l/s, die Strömungsgeschwindigkeit der Luft an der Eintrittsöffnung 41 bspw. 2-40 m/s. Im Innenvolumen 21 bildet sich eine Luftströmung, die das Garn in Zusammenwirkung mit der Innenwand 31 zu einem Knäuel verknäuelt. Wegen der nach oben weisenden Neigung des Eintrittsstutzens 4 und der Eintrittsöffnung 41 wird die Verknäuelung hauptsächlich, aber nicht notwendigerweise ausschliesslich, im oberen Teil des Behälters 2 stattfinden. Die eingeblasene Luft entweicht aus dem Innenvolumen 21 zuerst zur Achse a hin und dann, in einer Umgebung der Achse a, nach oben und/oder nach unten, durch die obere Öffnung 23 bzw. die Austrittsöffnung 22. Es ist vorteilhaft, wenn die obere Öffnung 23 eine grössere Fläche hat als die Austrittsöffnung 22, damit der Hauptteil der Luft aus dem Innenvolumen 21 nach oben entweicht und so eine nach oben gerichtete Kraft auf das zu verknäuelnde Garn ausübt.

Auf einem oberen Teil des Behälters 2 kann ein Ring 5 befestigt, bspw. aufgeklebt, sein, aus dem Luft im Wesentlichen horizontal in Richtung der oberen Öffnung 23 geblasen wird. Der Ausstoss dieser Luft kann z. B. radial nach innen, zur Achse a hin, tangential an den die obere Öffnung 23 begrenzenden Kreis, oder in einer anderen Richtung erfolgen. Er kann kontinuierlich oder stossweise, aus einer Richtung oder aus mehreren Richtungen erfolgen. Diese Lufteinblasung verhindert, dass das Garn mit der aus der oberen Öffnung 23 entweichenden Luft aus dem Torusteil herausgetragen wird. Die in den Figuren 1(a) und 1(b) sichtbaren Öffnungen 51 am äusseren Umfang des Rings 5 dienen der Zuleitung von Luft durch separate (nicht eingezeichnete) Leitungen. Alternativ kann diese Luft von der durch den Eintrittsstutzen 4 eingeblasenen Luft abgezweigt werden.

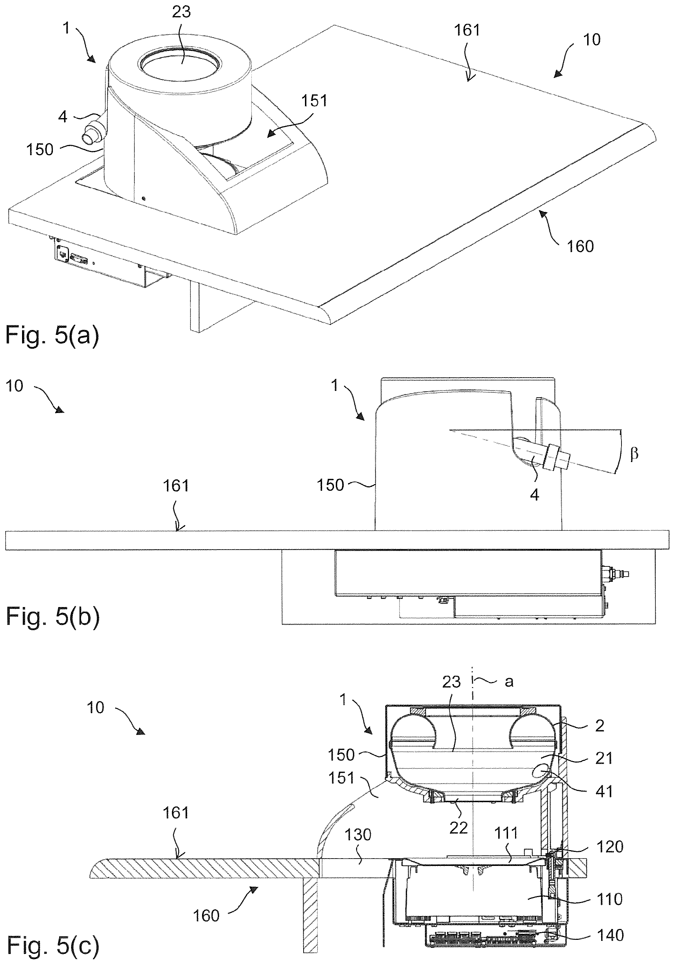

Wenn sich der gesamte zu verknäuelnde Garnabschnitt im Innenvolumen 21 befindet, wird die Luftströmung abgestellt. Dann fällt der Garnknäuel nach unten, auf den unteren Teil 33 der Innenwand 31. Wegen der Neigung des unteren Teils 33 der Innenwand 31 zur Austrittsöffnung 22 hin nach unten rollt und/oder gleitet der Knäuel aufgrund der Gravitationskraft auf dem geneigten Teil 33 bis zu einer Austrittsöffnung 22 und fällt durch diese aus dem Innenvolumen 21 heraus auf einen Wägeteller 111 einer Waage 110 (siehe Figur 5).

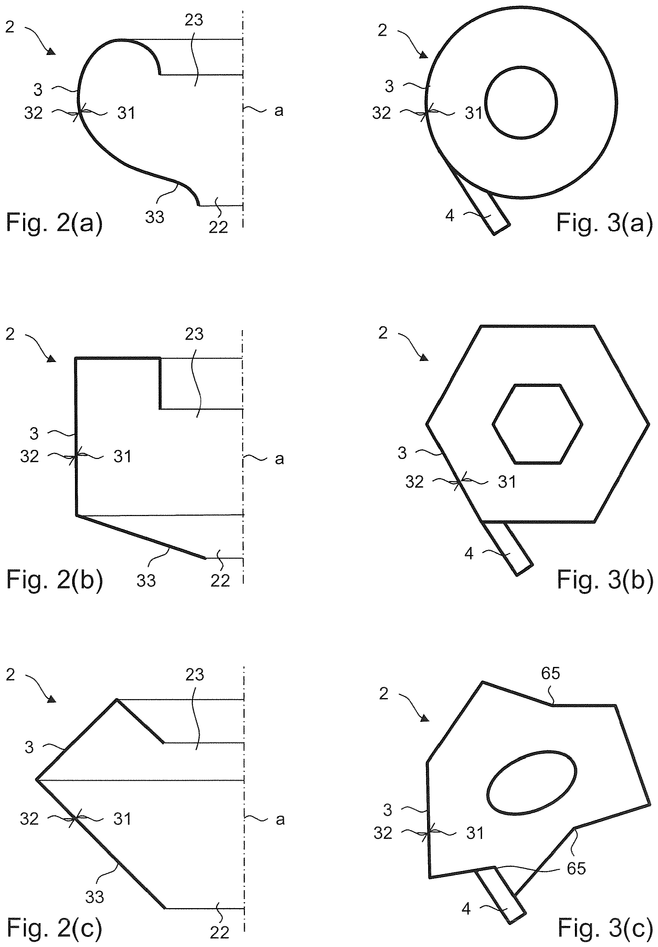

**Figur 2** zeigt schematisch Querschnitte von verschiedenen Ausführungsformen von Behältern 2 der erfindungsgemässen Vorrichtung 1 zur Verknäuelung von Garn. Wegen der Kreissymmetrie der hier gezeigten Ausführungsformen genügt es, jeweils nur eine Hälfte des Behälters 2 darzustellen. Die Ausuhrungsform von **Figur 2(a)** entspricht ungefähr derjenigen der Figur 1. Die **Figuren 2(b) und (c)** sollen illustrieren, dass auch andere Formen von Behältern 2 als ein Teil eines eingebetteten Torus zur Erfindung gehören. So sind Querschnitte mit konvexen oder konkaven Ecken durchaus möglich.

**Figur 3** zeigt schematisch Draufsichten von verschiedenen Ausführungsformen von Behältern 2 der erfindungsgemässen Vorrichtung 1 zur Verknäuelung von Garn. Diese sind unabhängig von den in Figur 2 gezeigten Ausführungsformen. Die Ausführungsform von **Figur 3(a)** entspricht ungefähr derjenigen der Figur 1. Die Figuren 3(b) und (c) sollen illustrieren, dass der Behälter 2 nicht kreissymmetrisch zu sein braucht. Der Behälter 2 von **Figur 3(b)** hat z. B. eine sechsfache Rotationssymmetrie, d. h. bildet in der Draufsicht ein regelmässiges Sechseck. Beim Behälter 2 von **Figur 3(c)** ist in der Draufsicht gar keine Symmetrie vorhanden. Der Behälter 2 hat in der Draufsicht die Form eines Vielecks mit (vom Innenvolumen 21 aus betrachtet) konkaven Ecken und konvexen Ecken 65.

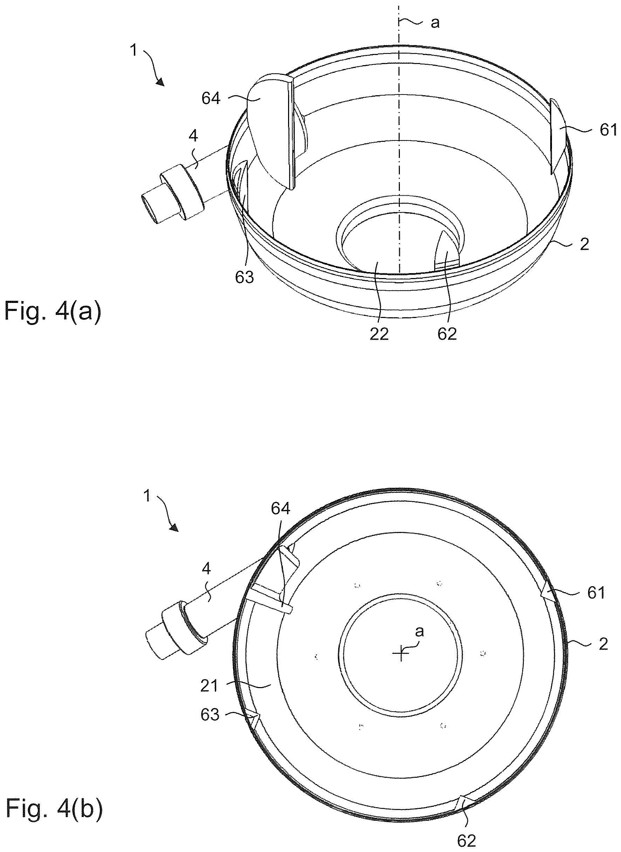

In der Ausführungsform von **Figur 4** weist die Innenwand 31 des kreissymmetrischen Behälters 2 mehrere in das Innenvolumen 21 hinein ragende Vorsprünge 61-64 auf. Diese haben die Aufgabe, als Schikanen die Bildung einer laminaren Luftströmung entlang der Innenwand 31 zu stören. Eine solche laminare Luftströmung ist deshalb unerwünscht, weil in ihr das eingeschossene Garn statt eines "chaotischen" Knäuels einen ringförmigen Wickel bilden kann, der das Innenvolumen 21 nicht durch die Austrittsöffnung 22 verlassen kann. Die Vorsprünge 61-64 können starr oder flexibel ausgebildet sein. Im letzteren Fall werden sie durch die Luftströmung in Strömungsrichtung gebogen und durch ihre eigene Elastizität wieder zurückgestellt. Dieser Vorgang wiederholt sich in schneller Abfolge, so dass eine regelmässig schwingende oder chaotisch flatternde Hin- und Herbewegung entsteht, durch welche wiederum die Luftströmung gestört wird. Die Vorsprünge 61-64 können regelmässig oder unregelmässig auf der Innenwand verteilt sein. Sie können untereinander identisch oder unterschiedlich sein. Die konvexen Ecken 65 in der Ausführungsform des Behälters 2 gemäss Figur 3(c) können auch als erfindungsgemässe Vorsprünge mit der oben beschriebenen Störfunktion betrachtet werden.

Die **Figur 5** zeigt eine Ausführungsform der erfindungsgemässen Vorrichtung 10 zum Wägen von Garn, welche die oben beschriebene erfindungsgemässe Vorrichtung 1 zur Verknäuelung von Garn beinhaltet, und zwar (a) in einer perspektivischen Ansicht, (b) in einer Seitenansicht und (c) in einem Längsschnitt. Unterhalb der Vorrichtung 1 zur Verknäuelung von Garn befindet sich eine Waage 110, vorzugsweise eine Präzisionswaage mit einem Wägeteller 111. Weder die Waage 110 noch irgendeiner ihrer Bestandteile 111 sind Teil der Vorrichtung 1 zur Verknäuelung von Garn. Dementsprechend sind die Vorrichtung 1 und die Waage 110 voneinander vertikal beabstandet, im Ausführungsbeispiel von Figur 3 um ca. 90 mm.

Wie oben beschrieben, fällt nach Abschluss der Knäuelbildung der Garnknäuel aufgrund der Gravitationskraft auf den Wägeteller 111. Dort wird er von der Waage 110 gewogen. Nach Beendigung der Wägung kann der Garnknäuel mittels mindestens einer Luftdüse 120, die zu diesem Zweck betätigbar ist, vom Wägeteller 111 weggeblasen werden. Der Garnknäuel wird vorzugsweise zu einer Abfallöffnung 130 geblasen, die zu einem darunter liegenden (nicht eingezeichneten) Abfallbehälter führt. Eine entsprechende Begrenzung, die durch die Verschalung 150 gebildet wird, dient als Anschlag für den Garnknäuel.

Die Vorrichtung 10 zum Wägen von Garn beinhaltet ferner Mittel 140 zur Steuerung des Messverfahrens und zur Auswertung der Messresultate. Die Steuerung umfasst das An- und Abstellen der Luft zum Einschiessen des Garns in das Innenvolumen 21, das An- und Abstellen der aus dem Ring 5 geblasenen Luft, die Messung der Länge des Garnabschnittes, das Schneiden des Garns, das Wägen des Garnknäuels und/oder das Wegblasen des Garnknäuels. Die Auswertung der Messresultate umfasst die Berechnung der Garnnummer aus der Länge und der Masse bzw. dem Gewicht des Garnabschnittes. Im Ausführungsbeispiel der Figur 3 befinden sich die Mittel 140 zur Steuerung des Messverfahrens und zur Auswertung der Messresultate unterhalb der Waage 110.
Die Vorrichtung 1 zur Verknäuelung von Garn kann mit einer Verschalung 150 versehen sein. Es ist Vorteilhaft, wenn die Verschalung 150 eine Öffnung 151 freilässt, durch welche eine Bedienungsperson auf den Wägeteller 111 blicken und notfalls zugreifen kann, um z. B. allfällige nicht weggeblasene Knäuel manuell zu entfernen, um den Wägeteller 111 zu reinigen etc. Die Vorrichtung 10 zum Wägen von Garn kann in einem Möbel 160 eingelassen sein, in dem oder unter dem sich der oben erwähnte Abfallbehälter befindet. Das Möbel 160 weist vorzugsweise eine ebene, horizontale Arbeitsfläche 161 auf, auf welche u. a. ein (nicht dargestellter) Bildschirm gestellt werden kann.

Selbstverständlich ist die vorliegende Erfindung nicht auf die oben diskutierten Ausführungsformen beschränkt. Bei Kenntnis der Erfindung wird der Fachmann weitere Varianten herleiten können, die auch zum Gegenstand der vorliegenden Erfindung gehören.

### BEZUGSZEICHENLISTE

- 1: Vorrichtung zur Verknäuelung von Garn

- 2: Behälter
- 21: Innenvolumen des Behälters
- 22: Austrittsöffnung des Behälters
- 23: obere Öffnung des Behälters

- 3: Wand des Behälters
- 31: Innenwand des Behälters
- 32: Aussenwand des Behälters
- 33: unterer Teil der Innenwand

- 4: Eintrittsstutzen
- 41: Eintrittsöffnung in der Innenwand

- 5: Ring auf dem Behälter
- 51: Öffnungen am äusseren Umfang des Rings

- 61-65: Vorsprünge an Innenwand

- 10: Vorrichtung zum Wägen von Garn

- 110: Waage
- 111: Wägeteller

- 120: eine oder mehrere Luftdüsen

- 130: Abfallöffnung

- 140: Steuer- und Auswertemittel

- 150: Verschalung der Vorrichtung zur Verknäuelung von Garn
- 151: Öffnung in der Verschalung

- 160: Möbel
- 161: Arbeitsfläche

- a: Symmetrieachse des kreissymmetrischen Innenvolumens

- α: Neigungswinkel des unteren Teils der Innenwand nach unten gegen die Horizontale
- β: Neigungswinkel der Eintrittsöffnung nach oben gegen die Horizontale

## Patentansprüche

1. Vorrichtung (1) zur Verknäuelung eines Garns, beinhaltend
einen Behälter (2) mit einem Innenvolumen (21), welches in vertikaler Richtung durch eine Innenwand (31) teilweise sowie in horizontaler Richtung durch die Innenwand (31) vollständig nach aussen begrenzt ist, wobei in der Innenwand (31) eine Eintrittsöffnung (41) angebracht ist, durch welche das Garn mit einer Luftströmung in das Innenvolumen (21) einschiessbar ist,
**dadurch gekennzeichnet, dass**
die Innenwand (31) starr ausgebildet ist,
das Innenvolumen (21) eine nach unten offene Austrittsöffnung (22) für einen im Innenvolumen (21) gebildeten Garnknäuel aufweist und
ein unterer Teil (33) der Innenwand (31) entlang des gesamten Umfangs der Austrittsöffnung (22) zur Austrittsöffnung (22) hin nach unten geneigt ist.

2. Vorrichtung (1) nach Anspruch 1, wobei ein Neigungswinkel (α) des unteren Teils (33) der Innenwand (31) gegen die Horizontale nicht grösser ist als -10°, bspw. zwischen -30° und -10° liegt und vorzugsweise -15° beträgt.

3. Vorrichtung (1) nach Anspruch 1 oder 2, wobei das Innenvolumen (21) um eine vertikale Achse (a) kreissymmetrisch ist.

4. Vorrichtung (1) nach Anspruch 3, wobei die Innenwand (31) im Wesentlichen die Form eines Teils eines eingebetteten Torus hat,
der durch einen in einer horizontalen Ebene liegenden Kreis definiert ist und dessen Achse (a) vertikal verläuft,
der nach innen offen ist und
dessen unterer Teil (33) zur Austrittsöffnung (22) hin nach unten geneigt ist.

5. Vorrichtung (1) nach einem der vorangehenden Ansprüche, wobei der Behälter (2) eine zentrale, nach oben offene Öffnung (23) aufweist, welche ein Ausströmen von Luft aus dem Innenvolumen (21) nach oben ermöglicht.

6. Vorrichtung (1) nach Anspruch 5, wobei die Fläche der nach oben offenen Öffnung (23) grösser ist als die Fläche der Austrittsöffnung (22).

7. Vorrichtung (1) nach einem der vorangehenden Ansprüche, wobei die Eintrittsöffnung (41) derart gestaltet und angeordnet ist, dass das Garn durch die Eintrittsöffnung (41) im Wesentlichen tangential zur Innenwand (31) in das Innenvolumen (21) einschiessbar ist.

8. Vorrichtung (1) nach Anspruch 7, wobei die Eintrittsöffnung (41) derart gestaltet und angeordnet ist, dass das Garn durch die Eintrittsöffnung (41) in einer nach oben geneigten Richtung in das Innenvolumen (21) einschiessbar ist, bspw. in einem Winkel (β) von 5° bis 25° und vorzugsweise von 15° gegen die Horizontale.

9. Vorrichtung (1) nach einem der vorangehenden Ansprüche, wobei die Innenwand (31) aus einem dünnen Material gebildet ist, das auch eine Aussenwand (32) des Behälters (2) bildet.

10. Vorrichtung (1) nach Anspruch 9, wobei ein Eintrittsstutzen (4), der in die Eintrittsöffnung (41) mündet, mit der Aussenwand (32) starr verbunden ist.

11. Vorrichtung (1) nach Anspruch 9 oder 10, wobei das die Innenwand (31) und die Aussenwand (32) bildende Material ein Metallblech, vorzugsweise Aluminiumblech, ist.

12. Vorrichtung (1) nach einem der vorangehenden Ansprüche, wobei die Innenwand (31) mindestens einen in das Innenvolumen (21) hinein ragenden Vorsprung (61-65) aufweist.

13. Vorrichtung (10) zum Wägen eines Garns, beinhaltend
eine Vorrichtung (1) zur Verknäuelung eines Garns und
eine Waage (110) zum Wägen eines durch die Verknäuelung gebildeten Garnknäuels,
**dadurch gekennzeichnet, dass**
die Vorrichtung (1) zur Verknäuelung eines Garns die Vorrichtung (1) nach einem der vorangehenden Ansprüche ist und
die Waage (110) unterhalb der Austrittsöffnung (22) angebracht ist.

14. Vorrichtung (10) nach Anspruch 13, zusätzlich beinhaltend mindestens eine Luftdüse (120) zum Wegblasen des Garnknäuels von der Waage (110).

15. Vorrichtung (10) nach Anspruch 13 oder 14, zusätzlich beinhaltend Mittel (140) zur Steuerung des Messverfahrens und zur Auswertung der Messresultate.

## Claims

1. An apparatus (1) for forming a ball of yarn, containing
a container (2) with an internal volume (21), which is outwardly bounded in the vertical direction partially by an internal wall (31) and in the horizontal direction completely by the internal wall (31), wherein an inlet opening (41) is provided in the internal wall (31) through which the yarn is injectable into the internal volume (21) by means of an air flow,
**characterized in that**
the internal wall (31) is formed in a rigid manner,
the internal volume (21) comprises a downwardly open outlet opening (22) for a yarn ball formed in the internal volume (21), and
a lower part (33) of the internal wall (31) is inclined downwardly towards the outlet opening (22) along the entire circumference of the outlet opening (22).

2. An apparatus (1) according to claim 1, wherein an angle of inclination (α) of the bottom part (33) of the internal wall (31) to the horizontal is not greater than -10°, and lies between -30° and -10° for example, and is preferably -15°.

3. An apparatus (1) according to claim 1 or 2, wherein the internal volume (21) is circular-symmetrical about a vertical axis (a).

4. An apparatus (1) according to claim 3, wherein the internal wall (31) substantially has the shape of a part of an embedded torus,
which is defined by a circle disposed in a horizontal plane and whose axis (a) extends vertically,
which is open to the inside, and
whose bottom part (33) is inclined downwardly towards the outlet opening (22).

5. An apparatus (1) according to one of the preceding claims, wherein the container (2) comprises a central, upwardly open opening (23) which allows an outflow of air from the internal volume (21) in the upward direction.

6. An apparatus (1) according to claim 5, wherein the area of the upwardly open opening (23) is greater than the area of the outlet opening (22).

7. An apparatus (1) according to one of the preceding claims, wherein the inlet opening (41) is formed and arranged in such a way that the yarn is injectable into the internal volume (21) through the inlet opening (41) substantially tangentially to the internal wall (31).

8. An apparatus (1) according to claim 7, wherein the inlet opening (41) is formed and arranged in such a way that the yarn is injectable into the internal volume (21) through the inlet opening (41) in an upwardly inclined direction, e.g. at an angle (β) of 5° to 25°, and preferably 15° to the horizontal.

9. An apparatus (1) according to one of the preceding claims, wherein the internal wall (31) is made of a thin material which also forms an outer wall (32) of the container (2).

10. An apparatus (1) according to claim 9, wherein an inlet nozzle (4), which opens into the inlet opening (41), is rigidly connected to the outer wall (32).

11. An apparatus (1) according to claim 9 or 10, wherein the material forming the internal wall (31) and the outer wall (32) is a sheet metal, preferably aluminium sheet.

12. An apparatus (1) according to one of the preceding claims, wherein the internal wall (31) comprises at least one projection (61 to 65) protruding into the internal volume (21).

13. An apparatus (10) for weighing a yarn, containing
an apparatus (1) for forming a ball of yarn and
a weighing machine (110) for weighing a ball of yarn formed by yarn ball formation,
**characterized in that**
the apparatus (1) for forming a ball of yarn is an apparatus (1) according to one of the preceding claims, and
the weighing machine (110) is arranged beneath the outlet opening (22).

14. An apparatus (10) according to claim 13, additionally containing at least one air nozzle (120) for blowing the ball of yarn away from the weighing machine (110).

15. An apparatus (10) according to claim 13 or 14, additionally containing means (140) for controlling the measuring method and for evaluating the measuring results.

## Revendications

1. Dispositif (1) pour le pelotage d'un fil, comprenant
un contenant (2) avec un volume intérieur (21), qui est délimité partiellement de l'extérieur dans le sens vertical par une paroi intérieure (31) et entièrement dans le sens horizontal par la paroi intérieure (31), une ouverture d'entrée (41) étant ménagée dans la paroi intérieure (31), par laquelle le fil peut être projeté dans le volume intérieur (21) avec un flux d'air,
**caractérisé en ce que**
la paroi intérieure (31) est rigide,
le volume intérieur (21) présente une ouverture de sortie (22) ouverte vers le bas pour une pelote de fil formée dans le volume intérieur (21) et
une partie inférieure (33) de la paroi intérieure (31) est inclinée vers le bas sur toute la circonférence de l'ouverture de sortie (22) en direction de l'ouverture de sortie (22).

2. Dispositif (1) selon la revendication 1, dans lequel un angle d'inclinaison (α) de la partie inférieure (33) de la paroi intérieure (31) par rapport à l'horizontale ne dépasse pas -10°, par exemple est compris entre -30° et -10° et est de préférence de -15°.

3. Dispositif (1) selon la revendication 1 ou 2, dans lequel le volume intérieur (21) est circulaire et symétrique autour d'un axe (a) vertical.

4. Dispositif (1) selon la revendication 3, dans lequel la paroi intérieure (31) a pour l'essentiel la forme d'une partie d'un tore intégré,
qui est défini par un cercle occupant un plan horizontal et dont l'axe (a) est vertical,
qui est ouvert vers l'intérieur et
dont la partie inférieure (33) est inclinée vers le bas en direction de l'ouverture de sortie (22).

5. Dispositif (1) selon l'une des revendications précédentes, dans lequel le contenant (2) présente une ouverture centrale (23) ouverte vers le haut, qui permet à l'air de s'échapper du volume intérieur (21) par le haut.

6. Dispositif (1) selon la revendication 5, dans lequel l'aire de l'ouverture (23) ouverte vers le haut est plus grande que celle de l'ouverture de sortie (22).

7. Dispositif (1) selon l'une des revendications précédentes, dans lequel l'ouverture d'entrée (41) est conformée et disposée de telle manière que le fil puisse être projeté à travers l'ouverture d'entrée (41) dans le volume intérieur (21) de façon sensiblement tangente de la paroi intérieure (31).

8. Dispositif (1) selon la revendication 7, dans lequel l'ouverture d'entrée (41) est conformée et disposée de telle manière que le fil puisse être projeté à travers l'ouverture d'entrée (41) dans le volume intérieur (21) en suivant une direction oblique vers le haut, par exemple sous un angle (β) de 5° à 25° et de préférence de 15° par rapport à l'horizontale.

9. Dispositif (1) selon l'une des revendications précédentes, dans lequel la paroi intérieure (31) est faite d'un matériau mince, qui forme aussi une paroi extérieure (32) du contenant (2).

10. Dispositif (1) selon la revendication 9, dans lequel un raccord d'entrée (4) débouchant dans l'ouverture d'entrée (41) est relié de façon rigide à la paroi extérieure (32).

11. Dispositif (1) selon la revendication 9 ou 10, dans lequel le matériau formant la paroi intérieure (31) et la paroi extérieure (32) est une tôle métallique, de préférence une tôle d'aluminium.

12. Dispositif (1) selon l'une des revendications précédentes, dans lequel la paroi intérieure (31) présente au moins une saillie (61-65) dépassant vers l'intérieur du volume intérieur (21).

13. Dispositif (10) pour le pesage d'un fil, comprenant
un dispositif (1) pour le pelotage d'un fil et
une balance (110) pour peser une pelote de fil formée par le pelotage,
**caractérisé en ce que**
le dispositif (1) pour le pelotage d'un fil est le dispositif (1) selon l'une des revendications précédentes et
la balance (110) est disposée en dessous de l'ouverture de sortie (22).

14. Dispositif (10) selon la revendication 13, comprenant en outre au moins une buse d'air (120) pour chasser la pelote de fil de la balance (110).

15. Dispositif (10) selon la revendication 13 ou 14, comprenant en outre des moyens (140) pour commander la mesure et analyser ses résultats.
